# EUROPEAN PATENT SPECIFICATION

(11) **EP 2 960 650 B1**
(45) Date of publication and mention of the grant of the patent: **23.10.2019**
(21) Application number: 14754002.5
(22) Date of filing: 19.02.2014
(51) Int. Cl.: G01N 33/53

(54) **ANALYTICAL IMMUNOSENSOR DEVICE AND METHOD FOR CONSTRUCTING SAME**
ANALYTISCHE IMMUNSENSORVORRICHTUNG UND VERFAHREN ZUR HERSTELLUNG DAVON
DISPOSITIF ANALYTIQUE IMMUNODÉTECTEUR ET SON PROCÉDÉ DE CONSTRUCTION

(30) Priority: 19.02.2013 ES 201330220
(43) Date of publication of application: 30.12.2015
(73) Proprietor: Universidad de Alcalá, 28801 Alcala de Henares (Madrid) (ES)
(72) Inventor: DOMÍNGUEZ CAÑAS, Elena, 28801 Alcalá de Henares (Madrid) (ES); NARVÁEZ GARCÍA, Arantzazu, 28801 Alcalá de Henares (Madrid) (ES); JIMÉNEZ CENTELLES, Javier, 28801 Alcalá de Henares (Madrid) (ES)
(74) Representative: Pons
(86) International application number: PCT/ES2014/070121
(87) International publication number: WO 2014/128328

(56) References cited:
- US-A- 4 895 809
- US-A- 5 534 132
- JAVIER JIMENEZ ET AL: "Quasi-reagentless electrochemical inmunosensors for the detection of quinolones", XV REUNION NACIONAL DE LA SOCIEDAD ESPAÑOLA DE QUIMICA ANALITICA, 19 July 2009 (2009-07-19), XP055164249,
- DOMÍNGUEZ, E. ET AL.: "Electrostatic Assemblies for Bioelectrocatalytic and Bioelectronic Applications.", ELECTROANALYSIS, vol. 18, no. 19-20, 2006, pages 1871-1878, XP55163127, cited in the application
- DOMÍNGUEZ, E. ET AL.: 'Electrostatic Assemblies for Bioelectrocatalytic and Bioelectronic Applications.' ELECTROANALYSIS vol. 18, no. 19-20, 2006, pages 1871 - 1878
- NARVAEZ, A. ET AL.: 'Reagentless biosensors based on self-deposited redox polyelectrolyte-oxidoreductases architectures.' BIOSENSORS AND BIOELECTRONICS vol. 15, no. 1-2, ISSN 0956-5663 pages 43 - 52
- CALVO E.J. ET AL.: 'Layer-by-layer electrostatic deposition of biomolecules on surfaces for molecular recognition, redox mediation and signal generation.' FARADAY DISCUSSIONS vol. 116, ISSN 1359-6640 pages 47 - 65
- LU , M. ET AL.: 'Flexible and disposable immunosensors based on layer-bylayer self-assembled carbon nanotubes and biomolecules.' 21ST INTERNATIONAL CONFERENCE ON MICRO ELECTRO MECHANICAL SYSTEMS, 2008 : MEMS 2008 ; vol. 13., pages 188 - 191
- PASTORINO, L. ET AL.: 'Development of a piezoelectric immunosensor for the measurement of paclitaxel.' JOURNAL OF IMMUNOLOGICAL METHODS vol. 313, 30 June 2006, pages 191 - 198

## Description

### OBJECT OF THE INVENTION

The invention as stated in the title of the present specification refers to an immunosensor as analytical device and to the method of construction of such analytical device.

More particularly, the object of the invention focuses on an immunosensor as analytical device based on the sequential deposition of self-assembled monolayers of polymers and affinity elements. This device can be applied to the detection and quantification of any analyte or target antigen in a liquid sample, enabling a displacement method of analysis, minimizing nonspecific adsorption and thus decreasing the detection limit. This is the case because the configuration of the device consists of a sensor surface firstly covered with polymer monolayers (redox or non-redox) and, on top of them, submonolayers of affinity elements are deposited; then, the affinity reaction takes place with the antigen, pseudo-antigen or the hapten labelled with (i) a protein, preferably an enzyme (redox or non-redox), with (ii) a compound or (iii) a nanoparticle resulting that the described method permits to develop "ad hoc" immunosensors, with a signal transduction by electrochemical, optical and/or piezoelectric means.

### FIELD OF THE INVENTION

The present invention falls into the industry field dedicated to the production of analytical devices, being particularly focused on the field of sensors, probes, systems and detection methods and analyte quantitation.

### BACKGROUND OF THE INVENTION

Sensors and immunosensors are analytical devices becoming reliable and simple analytical tools, with the advantage of being cheap, portable, selective, easy to use and they require a few microliters of sample to determine a specific parameter.

Usually, a chemical sensor or a biosensor is any miniaturized device capable of responding unequivocally to a particular analyte within a complex sample. It consists essentially of two parts: the recognition element that selectively interacts with the analyte, and the transducer, enabling the conversion of this interaction into an analytical signal.

Meanwhile an immunosensor is a biosensor in which the biological component is an immunochemical. Generally, the targeted compound is the antigen (analyte) or the hapten, and the antibody is then attached to the transducer, although, in some other cases, a better response is obtained when the analyte, or its modified form, is attached to the transducer.

Recently, the design of biosensors has been implemented making use of microelectronics, nanotechnologies and biomaterials improving then, their analytical properties and resulting in a key tool in cases where a fast decision-making is required.

Its ability to provide real-time information, its simplicity, its use by non-specialized-personnel and their ability to continuous or discrete monitoring are characteristics that offer clear advantages over conventional analytical tools.

In this sense, there is a need of versatile systems that could be designed "ad hoc" and becoming then ready for market introduction. (Bio)Sensors based on heterofunctional platforms allow the identification and the quantification of compounds of different nature by means of biorecognition elements in combination with different transduction principles and the appropriate signal-treatment.

For the construction of the heterofunctional platforms the "layer by layer" technique (LbL) is the most relevant. In this technique, the multi-layer films are created by the alternate deposition of oppositely charged materials with intermediate washing steps. Multi-layer films and washing steps can be performed in many different ways, including dip coating, spin coating, spray coating and flow techniques. The layer-by-layer technique offers remarkable advantages over other methods of thin-film deposition mainly simplicity and low cost. A wide variety of materials can be deposited by LbL method including polyions, metals, ceramics, nanoparticles and biological molecules. An additional advantage is the high degree of control over the thickness of the film, which growths up linearly with the number of bilayers. Since each bilayer may represent a few nm, this method provides an easy control of the thickness of the overall structure.

The aim of the present invention is thus to develop a method for the construction of an analytical device including the integration of the support and the detection elements by their physical immobilisation over a nanostructured organized interface. This sensor architecture object of the present invention will allow, for example, the detection of specific analytes in food and agriculture, in biomedical and in environmental industry and with any type of transduction.

As the background to the state of the art, it should be mentioned that there are different patents that disclose records related to similar analytical devices, the most related being the following:
- Patent U.S. 2002/ 0137193 A1 26.09.2002 which refers to a test system for the detection of ligand-ligand receptor binding (first member and second member), which comprises coating the electrode with a cross-linked redox polymer comprising a binding agent, a substrate-generating enzyme (both covalently bound to the redox hydrogel) and the first member of the receptor-ligand pair through an affinity reaction or by covalent bonding. The complex formed, generates an electrical signal when a potential to the electrode so that the applied electrical signal generated is related with the presence or amount of the second member in the sample. The first member is immobilized in the redox polymer on the electrode. The redox polymer may be a poly(vinyl pyridine) (poly (4 - vinyl pyridine). Additionally such redox polymers can be found complexed with transition metals such as osmium.
- U.S. Patent 5534132 A 09.07.1996 describes an amperometric biosensor for the detection of affinity reactions. This is an electrode having its surface substantially covered with transducing film and comprising a three-dimensional polymeric hydrogel in which the binding unit (SBU, selective binding unit) or its complement is immobilised. The selective binding agent allows introducing the additional component marked with the redox enzyme into the hydrogel, generating thereby an amperometric response through a redox electrocatalytic reaction of the enzyme substrate and the transduction of the catalytic activity of the enzyme, through the redox hydrogel to the electrode. The redox polymers used therein include polymers derived from quaternized poly (vinyl pyridine) with bromoethylamine bromide comprising osmium complexes, in order to form a cross-linkable hydrophilic redox polymer.
- The document PN - US2007092973 A1 discloses a film sensor for the detection of magnesium, comprising an indicator, a quaternary ammonium salt, imidazole or phosphorus, a surfactant and an acid. Among the chemicals that disclose the invention, a polymer positively charged, polyethyleneimine, and several crosslinking agents, polyethylene glycols derivatives are described.
- The document PN - WO0130495 A1 discloses an ion exchange resin in which appears as surface crosslinking agents, ethylene glycol diglycidyl ether and polyethylenimine, among others. However, its use is not mentioned as amperometric sensor for the detection of magnesium ions.

As far as scientific publications are concerned, the following papers are relevant:
- Reagentless biosensors based on redox polyelectrolyte-oxidoreductases self-deposited architectures. ARANTZAZU NARVAEZ ET AL. BIOSENSORS & BIOELECTRONICS vol. 15, 2000, pages 43-52. This paper describes the construction of biosensors based on self-assembled polyelectrolites multilayers by the LbL (Layer by Layer) technique on the negatively charged gold electrode. It also uses a redox polymer comprising Osmium.
- Electrostatic bioelectrocatalytic and assemblies for bioelectronic applications. ELENA DOMINGUEZ ET AL. Electroanalysis vol 18, 2006, pages 1871-1878, dealing with the use of LbL (Layer by Layer) assembly technology of electrolytes for the transduction of affinity and catalytic events.
- Surface charge effects on the redox switching of LbL self -assembled redox polyelectrolyte multilayers. MARIO E. TAGLIAZUCCHI, ERNERTO J. CALVO. JOURNAL OF ELECTROANALYTICAL CHEMISTRY, vol.599, 2007, pages 249-259. This paper describes some studies carried out of the effect of the surface charge on electron transfer and the charge propagation into the redox polyelectrolyte multilayers self-assembled by the LbL (layer by Layer) technique.
- Document entitled *"*Quasi-reagentless electrochemical inmunosensors for the detection of quinolones", by Javier Jimenez et al. ", XV REUNION NACIONAL DE LA SOCIEDAD ESPAÑOLA DE QUIMICA ANALITICA (19/7/2009), discloses a novel quasi-reagentless amperometric immunosensors based on multilayered heterofunctional assemblies for the detection of quinolones. The detection is based on the displacement of the labelled immunoagent by the presence of a simple rendering a concomitant signal decrease.

According with the literature found, it can be seen that there are in the state of the art, electrochemical immunosensors based on self-assembled polyelectrolite monolayers, as well as different types of amperometric sensors, however, none of that inventions and cited documents, taken alone or in combination, describes specifically, the technical characteristics and the constitutive features of the analytical immunosensor device and the method of construction of the present invention, as claimed.

### SUMMARY OF THE INVENTION

The present invention sets out the development of an analytical device as a universal support for the construction of quasi-reagentless and/or reagentless immunosensors. The invention also relates to a method for the construction of immunosensors based on sequential deposition of polymer monolayers and affinity elements, resulting in self-assembled sensor architectures. The main characteristic of this construction is the deposition of a small amount of affinity elements which allows the detection of the target analyte, using a displacement assay.

More specifically, the object of the invention is an analytical device comprising monolayers of polymers (redox or non-redox) covering the sensor surface, upon which, submonolayers of affinity elements are deposited, to further allow the affinity reaction with the bioconjugate (pseudo-antigen, hapten or antigen labelled with a tracer, tracer that can be a molecule, biomolecule or nanoparticle. This tracer results in a measurable signal).

These structures allow the development of "ad hoc" immunosensors, enabling electrochemical, optical, and/or piezoelectric transduction. Thus, the developed sensor platform allows to design electrochemical sensors (including amperometric, potentiometric, conductimetric and impedimetric sensors), optical sensors (based on optical fibres, surface plasmon resonance, evanescent wave sensors), and piezoelectric (including surface acoustic wave-based sensors), among others. Their applications are focussed, for example, to the detection of target analytes in environmental, food and clinical areas.

Therefore, the present invention describes a universal method for the construction of immunosensors based on electrostatic interactions, by the sequential deposition of the different elements.

Thus, the device of the invention allows, as a clear advantage, the detection of an analyte directly in one step, without using any additional immunoreactive and in a time interval no longer than 30 minutes. This polymer architecture offers a nanostructured interface enabling the deposition of a submonolayer of the antibodies and the bioconjugate (the last one, bonded by its affinity reaction with the antibody). The developed analytical device enables a displacement format assay, minimizing nonspecific adsorption and therefore decreasing the detection limit. The sample (pre-treated or not) is directly incubated onto the developed device and then, after washing and substrate addition (if necessary), an electrochemical, optical or microgravimetric signal, or a combination thereof, is obtained.

It should be noted that the analytical device or sensing interface disclosed in the present invention, enables an optical, electrochemical and/or microgravimetric transduction, depending on the requirements of the analysis. Furthermore, the method for the construction of the analytical device disclosed in the invention allows the deposition of a submonolayer of any antibody and its corresponding bioconjugated and thus, the final device obtained can detect and quantify any targeted analyte or antigen, for which the antibody selected shows affinity.

In conclusion, the invention proposes the construction of a fast, portable and low cost immunosensor, resulting in a sensor architecture that advantageously, enables the analysis of a sample in a single incubation step without addition of any immunoreactive.

Therefore, a first aspect of the present invention relates to an analytical immunosensor device, comprising the following layers:
(a) a sensing interface;
(b) a layer of antibodies assembled on the cationic polymer layer at the sensing interface
(c) a bioconjugate bound to the antibody layer by affinity reaction; characterized in that the sensor interface (a) comprises the following sequentially assembled layers:
   (i) a modified sensor surface with a negatively charged layer that comprises the -X-(CH₂)_{y}-SO³⁻ group; wherein X is selected from the -S-, -NH- o -COO- and y has a value between 1 and 6, and
   a polymeric structure comprising a polymeric layer comprising a cationic polymer
   and wherein the cationic polymer layer of the polymer structure is repeated n times comprising between these cationic layers, a polymeric layer of an anionic polymer wherein n ranges from 1 to 10.

The term "Bioconjugate" refers in the present invention an antigen or a hapten with affinity for the antibody and labelled with a tracer. This tracer can be a compound, a nanoparticle or a biomolecule capable of producing an electrochemical, optical or gravimetric signal or appropriated combinations thereof, in order to obtain an analytical determination by a displacement format assay. The bioconjugate can also be a "pseudo- antigen", namely a modified antigen through functional groups for its labelling with tracers and, if required, allowing the control of the affinity constants. The bioconjugate can be combined with a tracer that can be a compound, a nanoparticle or a biomolecule capable of producing a detectable signal. The given bioconjugate to be used will depend on the target analyte, on the antibody chosen in the previous layer and, on the type of signal to be detected. This selection is a general knowledge for those skilled in the art.

The biomolecule capable of generating a detectable signal may be or comprise a protein, more specifically an enzyme (recombinant or not), or an enzymatic fraction or a protein without catalytic activity. The compound capable of producing a detectable signal may be or comprise a chromophore, a fluorophore, or a radioisotope.

The term "enzymatic fraction" refers in the present invention, a polypeptide chain with catalytic activity or not. In the present invention could be, without limitation Microperoxidase MP-11, apoenzymes.

Enzymes which can be used in the present invention would be, without limitation, peroxidases such as horseradish peroxidase (HRP), alkaline phosphatases or tyrosinases.

Non-catalytic proteins which can be used in the present invention would be, without limitation, bovine serum albumin (BSA), lactalbumin or ovalbumin.

Fluorophores which can be used in the present invention would be, without limitation, fluorescein and derivatives, rhodamine, dansyl chloride, coumarin derivatives or proteins such as the Green Fluorescent Protein (GFP).

Radioisotopes which can be used in the present invention would be, without limitation, ³H, ¹²⁵I o ¹⁴C.

Nanoparticles which can be used in the present invention would be, without limitation, gold or silver nanoparticles, quatum-dots, nanotubes, nanowires, dendrimers.

In a preferred embodiment, the sensor surface is modified with a negatively charged compound comprising the -X-(CH₂)_{y}-SO³⁻ group: wherein X is selected from the groups -S-, -NH- or -COO- , y has a value between 1 and 3, and more preferably X is -S- and even more preferably the negatively charged compound is MPS (Sodium 3-mercapto-1-propanesulfonate). The sensor surface may be any substrate depending on the type of immunosensor, preferably is a flat surface and may be of a metal, glass, quartz, ceramic, plastic, or screen-pinted materials, for example can be of gold, platinum, carbon or glassy carbon, which may be additionally modified with colloids of the same or any other material among others known by one skilled person in the art.

The polymeric architecture comprises a cationic polymer which can be redox or non-redox. These polymers are conductive polymers with groups that dissociate in aqueous solutions resulting into polymers charged with cations. The redox polymer may be formed by complexing osmium, cobalt or ruthenium compounds with bipyridyl, poly(1-vinyl imidazole), polyethylenimine, poly(4-vinyl pyridine) or with a copolymer thereof.

In a preferred embodiment the polymer structure comprises a redox polymer with osmium, ruthenium, or ferrocene derivatives, such as Poly(Vinyl Ferrocene) as redox centers, or a non-redox cationic polymer, branched or not, such as polyethyleneimine, or any quaternized polymer of poly (vinyl pyridine) with bromoethylamine.

In a most preferred embodiment the cationic polymer is a redox polymer derived from poly(vinylpyridine), poly(vinylpyridine)Os(bpy)₂Cl quaternized with bromoethylamine, wherein bpy is bipyridine.

Going into the main details and technical features of the invention, the sequential deposition of oppositely charged polymer allows a controlled deposition of antibody in such a way that the developed interface minimizes the nonspecific adsorption.

The cationic polymer layer of the polymer architecture is be repeated up to 10 times, that is, the polymeric architecture comprises up to 11 sequential layers self-assembled of the positively charged polymer, integrating between cationic layers a polymer layer of an anionic polymer.

The anionic polymer is a conducting polymer or polyelectrolyte, for example but without limitation to poly(styrene sulfonic acid) (PSS) or sodium polyacrylate. In a preferred embodiment the anionic polymer is PSS.

The nature of the antibodies that comprises the antibody layer or submonolayer will depend on the target analyte and thus, it will be related to the labelled antigen or bioconjugate. These antibodies may be monoclonal or polyclonal.

Another aspect of the present invention relates to a method for obtaining the device disclosed in the present invention, comprising:
(a) depositing a negatively charged compound comprising the X-(CH₂)_{y}-SO³⁻ group, wherein X is selected from the groups -SH, -NH₂ o -COOH, and y has a value of between 1 and 6, on a sensing surface;
(b) depositing a polymeric layer of a cationic polymer onto the negatively charged modified sensor surface obtained in step (a);
(c) depositing of an antibody layer onto the deposited layer obtained in step (b), and
(d) incubating of the specific bioconjugate on the antibody layer obtained in step(c),
and where the method further comprised the deposition of n repetitions of the cationic polymer layer comprising between said cationic layers the deposition of a polymeric layer of an anionic polymer, wherein n ranges from 1 to 10.

In a particular embodiment the device obtained in the step (d) by the method disclosed above, is stored in dry conditions and kept cool between 2-10 ° C.

Specifically, the analytical device is obtained by layer-by-layer self-deposition of oppositely charged polymers. This is achieved through chemisorption of a negatively charged compound, for example, of a mercapto-derivative negatively charged such as (HS-(CH₂)_{y}-SO³⁻) and more particularly MPS (Sodium 3-mercapto-1-propanesulfonate), on the sensor surface such as gold electrode or any other sensor surface as described above. A cationic polymer (redox or non-redox) is then deposited on top of the previous layer. This polymer can be polyaminated and/or poly (vinyl pyridine) derivatives offering a controlled deposition of an antibody submonolayer. Later on, the incubation is performed with the corresponding bioconjugate, which may be or comprise an enzyme (redox or non-redox), as peroxidase, alkaline phosphatase, tyrosinase, etc. The developed sensor can be stored dried and cooled, for a minimum period of 6 months before use.

Another aspect of the present invention, relates to a method, for qualitative and/or quantitative analysis of a target analyte by a displacement assay comprising:
(i) the incubation of the device disclosed in the present invention directly with the sample comprising the analyte, and
(ii) measuring the obtained signal.

In a preferred embodiment of the analysis method of the invention, the signal obtained may be a microgravimetric signal, or a resonance units change, an optical or an electrochemical signal, and be measured by quartz-crystal microbalance (QCM), by Surface Plasmon Resonance (SPR), or by optical or electrochemical transduction, respectively.

In another preferred embodiment of the analysis method, prior to the measurement step (ii), the sample after its incubation with the sensor surface is washed, mainly with water and a substrate is added. The tracer used into the bioconjugate will determine the type of substrate.

For such analysis, the sample should be in liquid form, therefore for the case of a solid sample, a sample-pretreatment should be performed prior to the analysis. Biological samples are preferably isolated samples and therefore the methods of analysis are *in vitro.*

In general terms, any analyte can be determined according to the described method and to the developed device of this invention. The analyte may be of chemical or biological nature and this will determine the antibody and bioconjugate layers, being selected among others, but not limited to antibiotic residues as sufonamides, for example sulfamethoxazole, sulfadiazine, sulfabenzamide, sulfapirazol, sulfapyridine or sulfaquinoxaline; fluoroquinolones, for example ciprofloxacin, ofloxacin, levofloxacin; aminoglycosides, such as streptomycin, neomycin, gentamicin, tobramycin, amikacin, netilmicin, or kanamycin; glucocorticoids, for example hydrocortisone, cortisone, prednisolone, prednisone, methylprednisolone, fludrocortisone, aldosterone, dexamethasone and betamethasone; mycotoxins such as aflatoxins, DON , ochratoxin, zearelenona, T2 toxin fumonisin or/HT2; seafood natural toxins such as histamine, domoic acid or okadaic acid; detection of food allergens such as almond, lupine, peanut, milk, egg, mustard or soy. In clinical diagnosis, it can be used for the detection of biomarkers of neuropathological diseases such as Aβ-1-40, Aβ-1-42, Aβ-1-17, Tau, a-synuclein, DJ1; biomarkers of ageing including oxidative stress and neuroinflamatory pathways such as isoprostanes ROS, ROS, S-100-B, IL-1,IL-6, IL-8, CHI3L1 (YKL-40). In metabolic pathway associated with neurodegenerative diseases such as kynurenic acid, quinolinic acid and kynurenine. Analytes listed above are examples and they do not limit the scope of protection of the present invention.

Another aspect of this invention relates to the use of the device of the invention for the qualitative and/or quantitative detection of target analytes in chemical and biological samples.

The qualitative or quantitative analysis or detection of the target analytes is particularly useful for diagnostic applications, food or water quality control, among other applications in food and environmental safety and control.

The detection of the target analyte is performed directly upon the incubation of the sample with the sensor. During incubation, the analyte displaces the bound bioconjugate and rendering then a detectable signal, for instance a microgravimetric signal or Resonance Units change, or well after washing and the addition of the appropriate substrate, the generation of an optical or electrochemical signal. A minimum non-specific adsorption is achieved by using the device of the present invention with a displacement assay; this non specific adsorption is significantly lower than that obtained with a traditional competitive assay method.

In order to demonstrate the versatility of the analytical device, of the invention three different types of transduction are described as examples:
In a first example, the analytical device of the invention may comprise a cationic polymer as a redox polymer derived from poly(vinylpyridine)Os(bpy)₂Cl quaternised with bromoethylamine being the antigen, hapten and pseudoantigen labelled with a peroxidase (HRP). The displacement of the bioconjugate bound to the antibody by the analyte, present in the sample, results in a decrease of the amperometric response after a final washing step. In this case, the addition of substrate offers an efficient electrochemical communication between the redox polymer and the labelling enzyme. This efficient transduction is due to the designed electrostatic interactions between the different polymer layers. The resulting signal from the electrocatalytic reduction of the substrate (H₂O₂) is inversely proportional to the concentration of analyte in the sample.

In a second example, an optical transduction is shown both by Surface Plasmon Resonance (SPR) as well as by spectrophotometry. In the first case (SPR) the polymer used is a cationic polymer (for instance, polyamine or a poly(vinylpyridine) partially quaternised with bromoethylamine, redox or noredox) and an antigen, hapten and pseudoantigen labelled with an enzyme or non-catalytic protein (for example BSA, catalytic enzymes such as HRP or alkaline phosphatase, among others). In this case, the displacement of the bioconjugate results in a change of Resonace units. The signal decrease corresponds to the amount of analyte present in the sample, in such a way that larger amounts of analyte render lower signals. The analysis is performed in a single step where the addition of any substrate is not needed, obtaining a response at real-time.

In the case of the spectrophotometric transduction scheme, the labelling enzyme is any enzyme that catalyzes the conversion of any substrate onto a coloured product, such as HRP or alkaline phosphatase. After sample incubation and a washing step, a substrate is added. The catalytic conversion on the substrate renders a coloured product responsible of the spectrophotometric signal; also in this case and as in the previous example, inversely proportional to the analyte concentration present in the sample.

Finally, in the third example the microgravimetric detection by QCM is shown. As in the case of the SPR technique, the cationic polymer may be redox or non-redox and the label may be a high molecular weight protein like BSA. The signal is obtained at real time and after incubation with the sample. This is so, because the analyte present in the sample displaces the bioconjugate and thus, producing a frequency shift proportional to the analyte concentration. Additionally when a HRP or alkaline phosphatase is used as a label for the bioconjugate, the signal can be amplified by the use of a specific substrate that precipitates upon catalytic reaction.

In summary, the analytical device of the invention based on layer-by-layer self-assembly of polymers and affinity elements immobilised onto a sensor surface, has the peculiarity of being configured from a polymeric architecture forming a structure on which a submonolayer of antibody is deposited. This antibody is bound by affinity to the biocongugate wherein this bioconjugate is labelled with a marker: a chemical or biological compound or nanoparticle capable of generating a measurable signal.

Meanwhile, the analyte can be detected (qualitative analysis) or quantified (i) by electrochemical means, when the polymer structure can be a redox polymer, the bioconjugate may comprise a peroxidase and the substrate may be H₂O₂, (ii) by using Quartz Crystal Microbalance (QCM), when the polymer may be a cationic polymer (redox or non-redox) and the bioconjugate may comprise a protein or a nanoparticle, (iii) by surface plasmon resonance (SPR), when the polymer may be a cationic polymer (redox or non-redox) and the bioconjugate may comprise a protein or a nanoparticle; and finally (iv) by optical transduction, when the polymer can be a cationic polymer (redox or non-redox) and the bioconjugate may comprise a peroxidase or alkaline phosphatase, or tyrosinase.

The electrochemical or spectrophotometric transduction may comprise an incubation step of a sample drop (pre-treated or not), during a time not shorter than 0.5 min, a washing step with water and furthermore, the addition of an appropriate substrate according to the label; the Quartz Crystal microbalance detection and the Surface Plasmon Resonance detection comprise solely the incubation of a sample drop (pre-treated or not) and thus quantification at real-time.

### DESCRIPTION OF THE FIGURES

In order to get a full description and to contribute to a better understanding of the characteristics of the invention, a set of figures is attached to the present specification as an integral part thereof, in which with an illustrative and nonlimiting character is described the following:
Figure 1 -. Schematic representation of the analytical device of the invention, showing the sensor interface based on layer-by-layer self-assembly and the displacement by the analyte.
Figure 2a -. Calibration curves of the amperometric immunosensor of the invention for antibiotic and corticosteroid residues in a buffer solution.
Figure 2b -. Calibration curves of the amperometric immunosensor of the invention for antibiotic and corticosteroid residues in a sample of milk.
Figure 2c.- Shows the storage stability of the amperometric immunosensor of the invention for four different analytes.
Figure 3 -. Calibration curve for sulfapyridine obtained with a SPR based optical immunosensor of the invention.
Figure 4 -. Calibration curve of the optic immunosensor of the invention for dexamethasone.
Figure 5 -. Typical calibration curve of the piezoelectric immunosensor for sulfapyridine.
Figure 6 -. Comparative calibration curves for the displacement immunosensor of the invention with a traditional competitive immunosensor.

### PREFERRED EMBODIMENT OF THE INVENTION

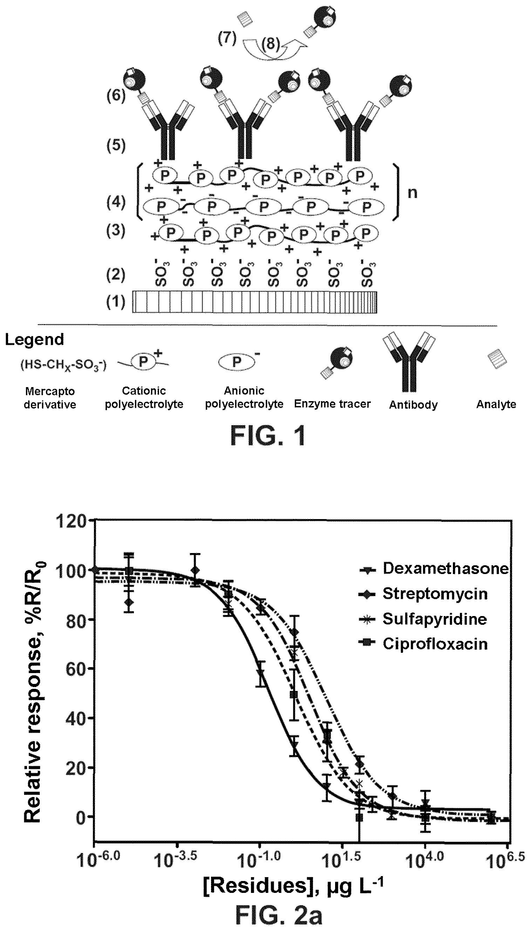

As shown in Figure 1, this invention describes a method for the construction of an immunosensor that includes a sensor surface modified with a monolayer of a mercapto-derivative negatively charged as (HS-(CH₂)_{y}-SO³⁻) for example MPS, on top of which a monolayer of a polyaminated cationic polymer, and/or derivatives of poly (vinyl pyridine), comprising or not, metal complexes particularly of osmium or ruthenium as redox centres, has been electrostatically deposited. (This cationic polymer layer can be repeated n times by alternating the sequential deposition with a negatively charged polymer layer, n can range from 1 to 10).

A submonolayer of antibodies is then deposited onto the cationic polymer layer, which is then ready for binding the bioconjugate labelled with an enzyme that may be redox or non-redox (peroxidase, alkaline phosphatase, tyrosinase, etc.). The developed sensing architecture can be stored dry and at 4° C for at least 6 months before use.

The detection of the target analyte is performed directly after incubation of the sample with the sensor, where the analyte displaces the bound bioconjugate from the antibody layer, rendering a microgravimetric signal or resonance units change, or alternatively after washing and the addition of the appropriate substrate, the generation of an optical or electrochemical signal.

Thus, the developed sensing structure allows:
- To use an optical, electrochemical and/or microgravimetric transduction, as required by the analysis.
- To assemble a submonolayer of any antibody so that the resulting device can detect and quantify any analyte or target antigen that exhibits affinity for the antibody chosen.
- To analyse the sample in a single incubation step, without adding any immunoreactive.
- In most cases, no sample pre-treatment is required.

To demonstrate the universality of the developed analytical device regarding the transduction scheme and the kind of antibody immobilised, three examples are shown allowing the detection and quantification of various analytes or target antigens:

Figure 1 Schematic representation of the analytical device of this invention. The method developed for obtaining the multilayer architecture is based on the sequential deposition of different polyelectrolytes by alternate immersion or by drop deposition of the sensor surface in the corresponding aqueous solutions. This method is described below:

Prior to use, the electrodes were cleaned (1):

The screen-printed gold electrodes were provided by Biosensor Technology GmbH (Berlin, Germany). The ceramic electrode support has dimensions of 2.5 cm long by 0.7 cm wide with a working electrode of 1 mm. These electrodes include a pseudo-reference Ag/AgCl electrode.

The electrodes were electrochemically treated by scanning at least 30 times from 0.1 to 1.5 V in a 5x10⁻⁵ M aqueous solution of H₂SO₄, in order to remove the inherent impurities of the screen-printing paste.

The first negatively charged monolayer was prepared by chemisorption of a mercapto-derivative on the cleaned gold surfaces. Therefore a self-assembled monolayer of a negatively charged mercapto-derivative (2) was deposited onto the cleaned gold surface (1). To do this, the sensor surface was incubated with 4 µL of a 1mM ethanolic solution 1:1 (EtOH/H₂O) of sodium 3-mercapto-1-propanesulfonate (MPS) for 12 h at 6°C (depicted as SO³⁻ in figure 1). After the incubation time, the electrodes were thoroughly washed with ethanol and deionised water.

Subsequently the cationic redox polymer is deposited by electrostatic interactions, a poly(vinylpyridine)Os(bpy)₂Cl redox polymer partially quaternised with bromoethylamine where bpy is bipyridine (P⁺) (3). Its deposition on gold/MPS electrodes was performed immersing the electrode into 2 mL of a redox polymer aqueous solution 1 mg mL⁻¹ for 2 h, protected from light and under controlled temperature of 27 °C. Then, the sensors were washed with deionized water and dried under nitrogen flow. This layer can be deposited as many times as needed by depositing an anionic polyelectrolyte (4) to reverse the charge, such as 250µM of an aqueous solution of poly(styrene sulfonic acid) (PSS). n can range from 1 to 10

The sensing structure also includes the antibodies that were deposited (5) from different solutions comprising the specific antibody at a concentration in the order of pg mL⁻¹ and depending on the given antibody. This structure, object of the invention (1-5), is common to all developed devices and to all transduction schemes used.

On top of the above structure, the specific bioconjugate (6) is bound by affinity interaction. The labelling of the bioconjugate depends on the desired transduction scheme.

The nature of the layers (5) and (6) is specific for each immunosensor targeting different analytes and it is exemplified as follows:
Sulfonamides: for this group of compounds the imunoreactives used were specific polyconal antibodies and a biocojugate synthesized according to the protocol described in the literature (Adrian, et al., J. Agric. Food Chem. 2009, 57, 385-394; Adrian, et al., Anal. Bioanal. Chem. 2008, 391, 1703-1712). In this case 2 µL of a 60 pg mL⁻¹ antibody aqueous solution were deposited for 2 h at 27°C. The sensors were then extensively rinsed with 0.1 M phosphate buffer pH 7.0.

Furthermore, the sensors comprising the antibody layer were incubated during 30 min at 27°C with 2 µL of a 2.5 mg L⁻¹ bioconjugate solution in 0.1 M phosphate buffer at pH 7.0. The sensors were then thoroughly washed with the same buffer and dried.
- Fluoroquinolones: for this group of compounds the imunoreactives (specific polyconal antibodies and bioconjugate) were provided by Applied Molecular Receptors Group (AMRG-CSIC) from the Spanish National Research Council (C.S.I.C.) in Barcelona and synthesized according to the protocol described in the literature (Adrian, et al., J. Agric. Food Chem. 2009, 57, 385-394; Adrian, et al., Anal. Bioanal. Chem. 2008, 391,1703-1712).

In this case 2 µL of a 10 pg mL⁻¹ antibody aqueous solution were deposited for 2 h at 27°C. The sensors were then extensively rinsed with 0.1 M phosphate buffer at pH 7.0.

Furthermore, the sensors comprising the antibody layer were incubated during 30 min at 27°C with 2 µL of a 2.1 mg L⁻¹ bioconjugate solution in 0.1 M phosphate buffer at pH 7.0. The sensors were then thoroughly washed with the same buffer and dried.

Aminoglycosides: for this group of compounds the imunoreactives (specific polyconal antibodies and bioconjugate) were provided by the Biomedicine group of Pharmacy department of King's College in London (UK) and synthesized according to the protocol described in the literature (Abuknesha, et al., Journal of Inmunological Methods, 2005, 306, 211-217).

In this case 2 µL of a 12 pg mL⁻¹ antibody aqueous solution were deposited for 2 h at 27°C. The sensors were then extensively rinsed with 0.1 M phosphate buffer at pH 7.0.

Furthermore, the sensors comprising the antibody layer were incubated during 30 min at 27°C with 2 µL of a 3.0 mg L⁻¹ bioconjugate solution in 0.1 M phosphate buffer at pH 7.0. The sensors were then thoroughly washed with the same buffer and dried.

Glucocorticoids: for this group of compounds commercial bioconjugates and polyclonal antibodies (anti-dexa ref.1098, dexa-HRP ref. C1098) supplied by the company Vitaltech (Barcelona, Spain) were used. The modified sensors were incubated with 2 µL of a 9 pg mL⁻¹ antibody aqueous solution for 2 h at 27°C. The sensors were then extensively rinsed with 0.1 M phosphate buffer at pH 7.0.

Furthermore, the sensors comprising the antibody layer were incubated during 30 min at 27°C with 2 µL of a 1.9 mgL⁻¹ bioconjugate solution in 0.1 M phosphate buffer at pH 7.0. The sensors were then thoroughly washed with the same buffer and dried.

During the assay, the displacement of the bioconjugate (6) by the presence of the analyte or target antigen (7) allows the detection and the quantification of trace and ultra-trace levels of certain analytes.

In the case of immunosensors with amperometric detection, the bioconjugate comprises a redox enzyme, in this case HRP (horseradish peroxidase). After the addition of H₂O₂ a cathodic current is generated being proportional to the remaining peroxidase in the immunosensor. The target analyte (7) in the sample will displace the bioconjugate rendering a decrease on the electrochemical response proportional to the concentration of the target analyte. This assay allows the detection of the analyte without sample pre-treatment.

The analytical procedure includes the incubation of the different samples with the electrode architecture previously described. After no more than 30 minutes of incubation, the electrode surfaces were washed with deionized water and placed in a substrate solution, resulting in immediate amperometric response.

The amperometric signals of the selective immunosensors were obtained through an AUTOLAB analyzer (Eco Chemie BV, Netherlands) integrated with software GPES 4.9. The analysis of the calibration curves was performed with GraphPad Prism TM (GraphPad Software Inc., San Diego, California, USA) software.

The developed immunosensors comprising the described architectures were used, among others, for the antibiotic and glucocorticoid residues detection in milk, by using a competitive displacement immunoassay with amperometric detection.

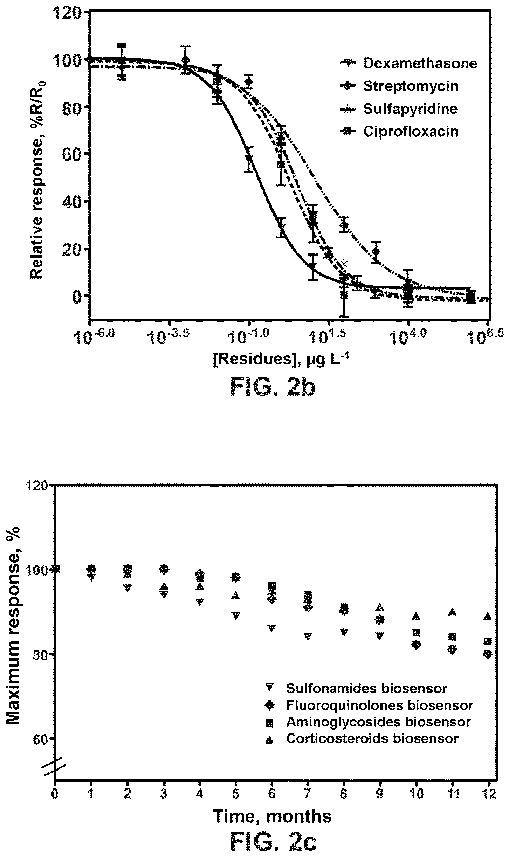

Figures 2a and 2b illustrate an application, as example, on screen-printed gold electrodes for the immunosensors described above. Figure 2a shows the results of calibration curves for antibiotic and corticosteroids residues in buffer solution. While Figure 2b shows the results of calibration curves for antibiotic and corticosteroid residues in milk samples. Experimental conditions were: 250 µM H₂O₂ in phosphate buffer 0.1 M pH 7.0 at 27°C. Eₐₚₚₗ: +0 mV vs. Ag/AgCI, KClₛₐₜ. The final volume into the cell was of 16 mL and the H₂O₂ solution was prepared from a "stock" solution protected from light.

Both figures show the dependence of the detectable concentration of analyte present in buffer aqueous solutions (Figure 2a) and in doped milk samples (Figure 2b), on the amperometric response changes. As shown in the figures, different analytes including dexamethasone, sulfapyridine, ciprofloxacin and streptomycin were detected. Figure 2c shows the storage stability of the developed devices. In order to verify their stability, the described immunosensors (comprising all layers) were dried and stored for 12 months at 4° C. Maximum amperometric response was measured for establishing their stability.

For Surface Plasmon Resonance based measurements (SPR), the sensing architecture (1-6) described above, was built up in flow conditions at 10 µL min⁻¹. To achieve this, successive injections of antibody (5) dissolved in acetate buffer pH 4.5 during 15 minutes were made. The biocongugate (6) comprising a protein (in this case the protein is a HRP) was dissolved in 0.1 M phosphate buffer pH 7 and successive injections were made for 10 minutes period on which no further changes on the resonance units were detectable.

Upon injection of analyte solution (7), the displacement of the bioconjugate occurs and this resulting in a decrease of resonant units related with the target analyte concentration. This assay allows detecting the target analyte without sample pre-treatment and with no additional use of any reagent ("reagentless" immunosensor).

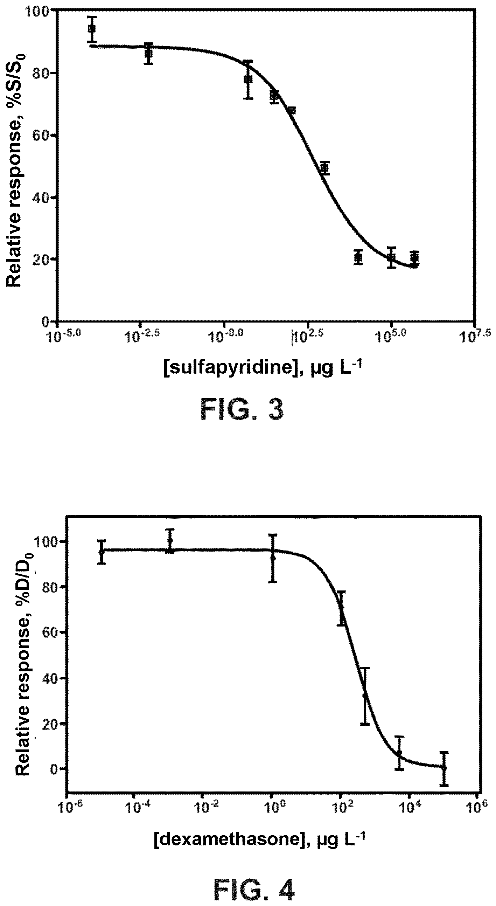

Figure 3 shows a calibration curve for sulfapyridine obtained with a SPR based optical immunosensor. The analyte in this case sulfapyridine was injected at various concentrations (from 0.01 µg L⁻¹ to 200 mg L⁻¹) dissolved in 0.1 M phosphate buffer pH 7.0 at a flow rate of 10 µL min⁻¹. The resonance unit's shift was monitored with a BIACORE X Software Set, (GE Healthcare, California USA). The analysis of the calibration curves was performed with GraphPad Prism TM (GraphPad Software Inc., San Diego, California, USA) software.

The graph shows the calibration curve for sulfapyridine in buffer with a SPR based optical immunosensor and obtained by competitive displacement assay. The figure illustrates that this format works regardless of the type of transduction used.

In the case of spectrophotometric transduction, the sensing structure (1-6) comprises an enzyme-labelled bioconjugate which catalyses the conversion of a substrate to a coloured product. The labelled bioconjugate solution in 0.1M phosphate buffer at pH 7.0 was deposited for 30 min at 27° C. The labelling enzyme may be HRP or alkaline phosphatase (in this assay the tracer used is HRP, Figure. 4). The sensors were thoroughly washed with the same buffer dried and stored at 4°C until analysis.

The developed immunosensors comprising the described architecture were tested for dexamethasone determination in buffer. After no more than 30 minutes of incubation with the sample, the electrode surfaces were washed with deionized water and placed in a substrate solution. This solution was spectrophotometrically measured at 650 nm in a UV-160A (Shimadzu, Columbia, USA) spectrophotometer. The analysis of the calibration curves was performed with GraphPad Prism TM (GraphPad Software Inc., San Diego, California, USA) software. This assay allows analyte detection without sample pre-treatment.

Figure 4 shows a typical calibration curve for dexamethasone in buffer obtained with an optical immunosensor. Experimental conditions: 0.01% of 3,3',5,5'-Tetramethylbenzidine (TMB) and 0,004% of H₂O₂ dissolved in citrate buffer pH 5.5. Signal readout obtained at 650 nm.

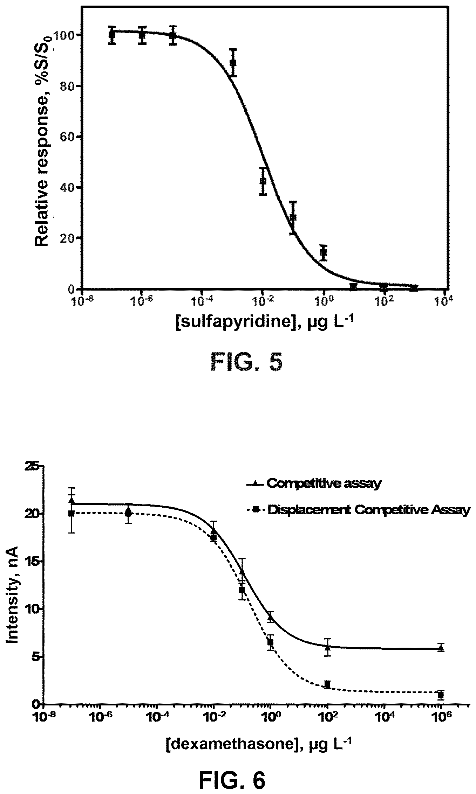

Finally and for microgravimetric based immunosensors (quartz crystal microbalance, QCM), the bioconjugate labelled with a protein (in this specific case, Figure 5, the tracer was a HRP) was bound to the sensing structure for a period of 30 min at 27° C and from a bioconjugate solution dissolved in 0.1M phosphate buffer at pH 7.0. The sensors were then thoroughly washed with the same buffer, dried and stored at 4°C until analysis. In this case 5 MHz AT-cut quartz crystal gold electrodes with a working area of 1.37 cm² were used for building up the sensing architecture. Prior to use, the quartz crystal electrodes were cleaned by immersion in piranha solution (70% H₂SO₄ : 30% H₂O₂) for 30 min, and then, treated with a freshly prepared saturated KOH solution for 30 min. To complete the cleaning process, the quartz crystals were later treated with a concentrated H₂SO₄ (18 M) during 30 min and a concentrated solution of HNO₃ (6M) for 15 min.

Once the electrodes were clean, the sensing surfaces were built up on electrostatic and affinity interactions following the same method and layers described previously. The quartz crystals were set in an MPS-550 probe and the frequency measured with a PM-710 plating monitor, all supplied by Maxtek Inc. The signal was recorded using a PMDATALG (Maxtek Inc.) software and processed with a personal computer.

The developed immunosensors comprising the described and optimised architectures were tested for sulfapyridine determination in buffer. The detection of the target analyte is performed on real time, directly after the sample incubation with the sensor, where the analyte displaces the bound bioconjugate, rendering a frequency shift proportional to the analyte concentration. In addition to this and if the tracer is a HRP or alkaline phosphatase, the signal may be amplified by the formation of an insoluble product catalytically generated. This assay allows detecting the target analyte without sample pre-treatment and with no additional use of any reagent ("reagentless" immunosensor).

A calibration curve for sulfapyridine in buffer with a QCM based immunosensor is shown in Figure 5. Experimental conditions: 100 µL of sample in 0,1 M phosphate buffer pH 7.0

Sufficiently described the nature of the present invention, as well as how to implement it, further explanations are not considered necessary to anyone skilled in the subject and pursuing the understanding of its scope and the advantages that arises from it. This invention, within its essentiality, may be performed in other embodiments that differ in detail from that indicated by way of examples, and which, are also covered by the protection claimed, provided they do not alter, change or modify its fundamental principle.

### Example: Comparison between a traditional competitive assay and a displacement assay

Figure 6 shows calibration curves of dexamethasone dissolved in 0.1 M phosphate buffer pH 7.0 for a traditional competitive assay format (▲) and for a displacement assay format (■). Error bars indicate standard deviations of 15 measurements per concentration.

The first one (▲), was obtained according to a conventional competitive assay procedure in which the bioconjugate and sample are previously mixed during the assay, being this mixture incubated with the sensor comprising immobilized antibody (1-5).

The second curve has been obtained following the competitive displacement assay (■), object of the invention, in which the bioconjugate is bound, by affinity reaction, to the antibody layer (1-6) being incorporated to the sensing architecture (with no need of mixing it with the sample). The only step is the incubation of the sample with the sensing structure for 30 min.

Later competitive assays were performed in both formats:
1. For the traditional competitive assay: In this case, 1.9 mg L⁻¹ of bioconjugate (dexa-HPR) was added to the sample. The mixture was incubated during 30 minutes at room temperature on the sensing structure comprising all the layer films until the antibody (anti-dexa) layer, (1) - (5) in Figure 1. After that time the sensor was thoroughly washed with deionised water.
2. For the competitive displacement assay: in this case, the sensor structure comprising the antibody layer (1-5) was incubated during 30 min at 27°C with 2 µL of 1.9 mg L⁻¹ bioconjugate solution (dexa-HRP) in 0.1 M phosphate buffer at pH 7.0. The sensors were thoroughly washed with the same buffer and dried.

The completed sensing architecture integrating the antibody and the bioconjugate, layers (1)-(6) in Figure 1, was incubated with the sample for 30 min. After that time the sensor was thoroughly washed with deionised water.

In both cases, the electrochemical measurements were carried out in a cell comprising 250 µM H₂O₂ in phosphate buffer 0.1 M pH 7.0 resulting in a cathodic current at 0 mV vs. Ag/AgCI KClₛₐₜ. The final volume into the cell was 16 mL and the H₂O₂ solution was prepared from a stock solution protected from light.

As shown in Figure 6, the comparison of these two formats assay results in a longer linear range as well as a lower non-specific adsorption for the curve performed by the displacement assay being this lowering in the order of 30%.

## Claims

1. Analytical immunosensor device comprising the following layers:
a) a sensing interface;
b) a layer of antibodies assembled on the cationic polymer layer at the sensing interface, and
c) a bioconjugate bound by affinity reaction to the antibody layer; **characterized in that** the sensor interface (a) comprises the following sequentially assembled layers:
(i) a modified sensor surface with a negatively charged layer that comprises the -X-(CH₂)_{y}-SO³⁻ group; wherein X is selected from the following groups: -S-, -NH- or -COO-, and y has a value between 1 and 6; and
(ii) a polymeric structure comprising a polymeric layer comprising a cationic polymer,
wherein
the cationic polymer layer of the polymer structure is repeated n times comprising between these cationic layers, a polymeric layer of an anionic polymer wherein n ranges from 1 to 10
and wherein
the bioconjugate is an antigen, a pseudo-antigen or a hapten with affinity for the antibody and labelled with a tracer, wherein the tracer is a compound, a nanoparticle or a biomolecule capable of producing a detectable signal.

2. Device according to claim 1, wherein the tracer comprises a non-catalytic protein, an enzyme, a chromophore, a fluorophore, a radioisotope, a nanoparticle, or any combination thereof.

3. Device according to claim 2, wherein the non-catalytic protein is BSA or the enzyme is selected from: HRP, tyrosinase or alkaline phosphatase

4. Device according to any of claims 1 to 3, wherein the sensor surface is selected from: metal, glass, quartz, ceramic material, plastic material, or screen-printed materials.

5. Device according to any of claims 1 to 4 wherein the negative charged layer of the sensor surface is S-(CH₂)_{y}-SO³⁻; and y has a value between 1 and 3.

6. Device according to any of claims 1 to 5 wherein the sensor surface is modified with sodium 3-mercapto-1-propanesulfonate.

7. Device according to any of claims 1 to 6 wherein the polymer structure comprises: a redox polymer with osmium, ruthenium or ferrocene derivatives as redox centres; or a non redox cationic polymer, branched or not, selected from: polyethyleneimine or any quaternized polymer from poly (vinylpyridine) with bromoethylamine.

8. Device according to any of claims 1 to 6 wherein the polymeric structure comprises poly(vinylpyridine)Os(bpy)₂Cl quaternized with bromoethylamine.

9. Device according to any of claims 1 to 8, wherein the anionic polymer is poly(sodium 4-styrenesulfonate).

10. Method for obtaining the device according to any of the preceding claims comprising:
a) Over a sensing surface, depositing a negatively charged compound comprising the X-(CH₂)_{y}-SO³⁻ group: wherein X is selected from the groups -SH, -NH₂ or -COOH, and y has a value of between 1 and 6;
b) depositing a polymeric layer of a cationic polymer onto the negatively charged modified sensor surface obtained in step (a);
c) depositing an antibody layer onto the deposited layer obtained in step (b), and
d) incubating the bioconjugate on the antibody layer obtained in step(c).
and where the method further comprised the deposition of n repetitions of the cationic polymer layer comprising between said cationic layers the deposition of a polymeric layer of an anionic polymer, wherein n ranges from 1 to 10.

11. Method for qualitative and/or quantitative analysis of an analyte by a displacement assay comprising:
(i) the incubation of the device according to any of claims 1 to 9 directly with the isolated liquid sample comprising the analyte, and
(ii) the measuring of the obtained signal.

12. Use of the device according to any of claims 1 to 9 for qualitative and/or quantitative detection of chemical and biological analytes in isolated samples.

13. Use according to claim 12 for diagnostic, food or environmental safety and control applications.

## Patentansprüche

1. Analytische Immunsensorvorrichtung, umfassend die folgenden Schichten:
a) eine Sensorschnittstelle;
b) eine Antikörperschicht, die auf der kationischen Polymerschicht an der Erfassungsschnittstelle angeordnet ist, und
c) ein Biokonjugat, das durch Affinitätsreaktion an die Antikörperschicht gebunden ist;
**dadurch gekennzeichnet, dass** die Sensorschnittstelle (a) die folgenden sequenziell angeordneten Schichten umfasst:
(i) eine modifizierte Sensoroberfläche mit einer negativ geladenen Schicht, welche die-X-(CH₂)_{y}SO³⁻-Gruppe umfasst; wobei X aus den folgenden Gruppen ausgewählt ist: -S-, -NH- oder -COO- und y einen Wert zwischen 1 und 6 aufweist; und
(ii) eine Polymerstruktur, die eine Polymerschicht umfasst, die ein kationisches Polymer umfasst,
wobei die kationische Polymerschicht der Polymerstruktur n-mal wiederholt ist, umfassend zwischen diesen kationischen Schichten eine Polymerschicht aus einem anionischen Polymer, wobei n im Bereich von 1 bis 10 liegt und wobei
das Biokonjugat ein Antigen, ein Pseudo-Antigen oder ein Hapten mit Affinität zu dem Antikörper ist und mit einem Tracer markiert ist, wobei der Tracer eine Verbindung, ein Nanopartikel oder ein Biomolekül ist, das ein nachweisbares Signal erzeugen kann.

2. Vorrichtung nach Anspruch 1, wobei der Tracer ein nicht-katalytisches Protein, ein Enzym, einen Chromophor, einen Fluorophor, ein Radioisotop, ein Nanopartikel oder eine beliebige Kombination davon umfasst.

3. Vorrichtung nach Anspruch 2, wobei das nicht-katalytische Protein BSA ist oder das Enzym ausgewählt ist aus: HRP, Tyrosinase oder alkalischer Phosphatase

4. Vorrichtung nach einem der Ansprüche 1 bis 3, wobei die Sensoroberfläche ausgewählt ist aus: Metall, Glas, Quarz, Keramikwerkstoff, Kunststoffwerkstoff oder im Siebdruckverfahren aufgebrachten Werkstoffen.

5. Vorrichtung nach einem der Ansprüche 1 bis 4, wobei die negativ geladene Schicht der Sensoroberfläche S-(CH₂)_{y}-SO³⁻ ist und y einen Wert zwischen 1 und 3 aufweist.

6. Vorrichtung nach einem der Ansprüche 1 bis 5, wobei die Sensoroberfläche mit Natrium-3-mercapto-1-propansulfonat modifiziert ist.

7. Vorrichtung nach einem der Ansprüche 1 bis 6, wobei die Polymerstruktur Folgendes umfasst: ein Redoxpolymer mit Osmium-, Ruthenium- oder Ferrocenderivaten als Redoxcenter; oder ein kationisches Nicht-Redox-Polymer, verzweigt oder nicht, ausgewählt aus Polyethylenimin oder einem quaternisierten Polymer aus Poly(vinylpyridin) mit Bromethylamin.

8. Vorrichtung nach einem der Ansprüche 1 bis 6, wobei die Polymerstruktur Poly(vinylpyridin)Os(bpy)₂Cl, quaternisiert mit Bromethylamin, umfasst.

9. Vorrichtung nach einem der Ansprüche 1 bis 8, wobei das anionische Polymer Poly(natrium-4-stryrolsulfonat) ist.

10. Verfahren zum Erhalten der Vorrichtung nach einem der vorhergehenden Ansprüche, umfassend:
a) Abscheiden einer negativ geladenen Verbindung über einer Sensoroberfläche, welche die X-(CH₂)_{y}-SO³⁻-Gruppe umfasst: wobei X aus den Gruppen -SH, -NH₂ oder -COOH ausgewählt ist und y einen Wert zwischen 1 und 6 aufweist;
b) Abscheiden einer Polymerschicht aus einem kationischen Polymer auf der negativ geladenen modifizierten Sensoroberfläche, die in Schritt (a) erhalten wurde;
c) Abscheiden einer Antikörperschicht auf der in Schritt (b) erhaltenen abgeschiedenen Schicht und
d) Inkubieren des Biokonjugats auf der in Schritt (c) erhaltenen Antikörperschicht,
und wobei das Verfahren ferner die Abscheidung von n Wiederholungen der kationischen Polymerschicht umfasst, die zwischen den kationischen Schichten die Abscheidung einer Polymerschicht eines anionischen Polymers umfasst, wobei n im Bereich von 1 bis 10 liegt.

11. Verfahren zur qualitativen und/oder quantitativen Analyse eines Analyten durch einen Verdrängungsassay, umfassend:
(i) die direkte Inkubation der Vorrichtung nach einem der Ansprüche 1 bis 9 mit der isolierten flüssigen Probe, die den Analyten umfasst, und
(ii) die Messung des erhaltenen Signals.

12. Verwendung der Vorrichtung nach einem der Ansprüche 1 bis 9 zum qualitativen und/oder quantitativen Nachweis chemischer und biologischer Analyten in isolierten Proben.

13. Verwendung nach Anspruch 12 zur Diagnose, Lebensmittel- oder Umweltsicherheit und für Kontrollanwendungen.

## Revendications

1. Dispositif immunocapteur analytique comprenant les couches suivantes :
a) une interface sensorielle ;
b) une couche d'anticorps assemblés sur la couche de polymère cationique à l'interface sensorielle, et
c) un bioconjugué lié par réaction d'affinité à la couche d'anticorps ;
**caractérisé en ce que** l'interface capteur (a) comprend les couches suivantes assemblées séquentiellement :
(i) une surface de capteur modifiée avec une couche chargée négativement qui comprend le groupe -X- (CH₂)_{y}-SO³⁻ ; où X est choisi parmi les groupes suivants : -S-, -NH- ou -COO-, et y a une valeur comprise entre 1 et 6 ; et
(ii) une structure polymère comprenant une couche polymère comprenant un polymère cationique,
dans lequel
la couche de polymère cationique de la structure de polymère est répétée n fois comprenant entre ces couches cationiques, une couche polymère d'un polymère anionique dans laquelle n est compris entre 1 et 10
et dans lequel
le bioconjugué est un antigène, un pseudo-antigène ou un haptène ayant une affinité pour l'anticorps et marqués avec un traceur, dans lequel le traceur est un composé, une nanoparticule ou une biomolécule capable de produire un signal détectable.

2. Dispositif selon la revendication 1, dans lequel le traceur comprend une protéine non catalytique, une enzyme, un chromophore, un fluorophore, un radioisotope, une nanoparticule, ou toute combinaison de ceux-ci.

3. Dispositif selon la revendication 2, dans lequel la protéine non catalytique est la BSA ou l'enzyme est choisie parmi : HRP, tyrosinase ou phosphatase alcaline.

4. Dispositif selon l'une quelconque des revendications 1 à 3, dans lequel la surface du capteur est choisie parmi : métal, verre, quartz, matériau céramique, matière plastique ou matériaux sérigraphiés.

5. Dispositif selon l'une quelconque des revendications 1 à 4, dans lequel la couche chargée négativement de la surface du capteur est S-(CH₂)_{y}-SO³⁻ ; et y a une valeur comprise entre 1 et 3.

6. Dispositif selon l'une quelconque des revendications 1 à 5, dans lequel la surface du capteur est modifiée avec du 3-mercapto-1-propanesulfonate de sodium.

7. Dispositif selon l'une quelconque des revendications 1 à 6, dans lequel la structure de polymère comprend : un polymère redox avec de l'osmium, des dérivés de ruthénium ou de ferrocène en tant que centres d'oxydo-réduction ; ou un polymère cationique non redox, ramifié ou non, choisi parmi : la polyéthylèneimine ou tout polymère quaternisé de poly(vinylpyridine) avec de la bromoéthylamine.

8. Dispositif selon l'une quelconque des revendications 1 à 6, dans lequel la structure polymère comprend la polyvinylpyridine)Os (bpy)₂Cl quaternisée avec la bromoéthylamine.

9. Dispositif selon l'une quelconque des revendications 1 à 8, dans lequel le polymère anionique est le poly(4-styrènesulfonate de sodium).

10. Procédé d'obtention du dispositif selon l'une quelconque des revendications précédentes, comprenant :
a) Sur une surface sensorielle, le dépôt d'un composé chargé négativement comprenant le groupe X- (CH₂)_{y}-SO³⁻ : dans lequel X est choisi dans les groupes -SH, -NH₂ ou -COOH, et y a une valeur comprise entre 1 et 6 ;
b) le dépôt d'une couche polymère d'un polymère cationique sur la surface de capteur modifiée chargée négativement obtenue à l'étape (a) ;
c) le dépôt d'une couche d'anticorps sur la couche déposée obtenue à l'étape (b), et
d) l'incubation du bioconjugué sur la couche d'anticorps obtenue à l'étape (c).
et où le procédé comprend en outre le dépôt de n répétitions de la couche de polymère cationique comprenant entre lesdites couches cationiques le dépôt d'une couche polymère d'un polymère anionique, où n est compris entre 1 et 10.

11. Procédé d'analyse qualitative et/ou quantitative d'un analyte par un test de déplacement comprenant :
(i) l'incubation du dispositif selon l'une quelconque des revendications 1 à 9 directement avec l'échantillon liquide isolé comprenant l'analyte, et
(ii) la mesure du signal obtenu.

12. Utilisation du dispositif selon l'une quelconque des revendications 1 à 9 pour la détection qualitative et/ou quantitative d'analytes chimiques et biologiques dans des échantillons isolés.

13. Utilisation selon la revendication 12 pour des applications diagnostique, de sécurité alimentaire et environnementale et de contrôle.
